(19) Europäisches Patentamt
European Patent Office
Office européen des brevets

(11) **EP 4 663 115 A1**

(12) **EUROPEAN PATENT APPLICATION**
published in accordance with Art. 153(4) EPC

(43) Date of publication:
**17.12.2025 Bulletin 2025/51**

(21) Application number: **24753375.5**

(22) Date of filing: **07.02.2024**

(51) International Patent Classification (IPC):
**A61B 5/055** (2006.01)          **G06T 1/00** (2006.01)
**G06T 1/40** (2006.01)

(52) Cooperative Patent Classification (CPC):
**A61B 5/055; G06T 1/00; G06T 1/20**

(86) International application number:
**PCT/JP2024/004071**

(87) International publication number:
**WO 2024/166932 (15.08.2024 Gazette 2024/33)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **07.02.2023 JP 2023016839**

(71) Applicants:
• **University Public Corporation Osaka**
  **Osaka-shi, Osaka 5360025 (JP)**

• **Iida Sangyo Co., Ltd.**
  **Musashino-shi, Tokyo 180-0022 (JP)**

(72) Inventors:
• **UEDA Daiju**
  **Osaka-shi, Osaka 558-8585 (JP)**
• **MORI Kazuhiko**
  **Musashino-shi, Tokyo 180-0022 (JP)**
• **MATSUMOTO Koichi**
  **Musashino-shi, Tokyo 180-0022 (JP)**

(74) Representative: **Mewburn Ellis LLP**
**Aurora Building**
**Counterslip**
**Bristol BS1 6BX (GB)**

(54) **MEDICAL IMAGE GENERATION METHOD AND DEVICE, ARTIFICIAL INTELLIGENCE MODEL TRAINING METHOD AND DEVICE, AND PROGRAM**

(57)    An object of the present invention is to generate a synthetic image which is similar to a positron emission tomography (PET) image from a magnetic resonance (MR) image of a patient. One embodiment of the present invention provides, a medical image generation method characterized by causing execution of the steps of: acquiring a magnetic resonance (MR) image of a particular patient suspected of having a certain disease; and generating, from the MR image of the particular patient, a synthetic image similar to a positron emission tomography (PET) image, by using a learned model obtained by learning an artificial intelligence model by using, as learning data, MR images of patients diagnosed to have the disease and the PET images corresponding to the MR images.

FIG. 6

**Description**

TECHNICAL FIELD

The present invention relates to a method, device and program for generating a synthetic image which is similar to a positron emission tomography (PET) image from a magnetic resonance (MR) image of a patient.

BACKGRAUND ART

C-Methionine Positron Emission Tomography (hereinafter referred to as MET-PET) is conducted to patients with suspected brain tumors to evaluate the accumulation of methionine in the areas of active amino acid metabolism. The MET-PET examination is useful for glioma grading, prognosis prediction, and delineation of tumor extent. Recent guidelines have supported the use of the MET-PET for the preoperative evaluation of patients with gliomas. However, the MET-PET examination requires a cyclotron and scanner, and therefore the facilities that can use the examination are limited, and not all patients with suspected gliomas can undergo the MET-PET examination. Furthermore, the MET-PET examination involves radiation exposure of the patient.

For gliomas, both methionine uptake and enhancement of contrast-enhanced T1-weighted images in magnetic resonance (CE-MR) examinations are reported to indicate cell proliferation and angiogenesis (Non-Patent Documents 1 to 4). This suggests that MET-PET and CE-MR features are correlated with each other.

In recent years, the image-to-image translation techniques based on deep learning have been developed (Non-Patent Documents 5 to 8). The image-to-image translation model involves the application of generative adversarial networks that can extract features between the images (Non-Patent Document 9). For example, the computed tomography images for attenuation correction have been generated from the $[^{18}F]$-2-fluoro-2-deoxy-D-glucose positron emission tomography (FDG-PET) images (Non-Patent Document 10), and the MR brain images have been generated from the brain FDG-PET images (Non-Patent Document 11).

PRIOR ART REFERENCE

Non-Patent Reference

Non-Patent Document 1: Langen KJ, Muhlensiepen H, Holschbach M, et al: Transport mechanisms of 3-[123I]iodo-alpha-methyl-L-tyrosine in a human glioma cell line: comparison with [3H]methyl]-L-methionine. J Nucl Med 41:1250-5, 2000

Non-Patent Document 2: Kracht LW, Friese M, Herholz K, et al: Methyl-[11C]-l-methionine uptake as measured by positron emission tomography correlates to microvessel density in patients with glioma. Eur J Nucl Med Mol Imaging 30:868-73, 2003

Non-Patent Document 3: Tynninen O, Aronen HJ, Ruhala M, et al: MRI enhancement and microvascular density in gliomas. Correlation with tumor cell proliferation. Invest Radiol 34:427-34, 1999

Non-Patent Document 4: Sadeghi N, Salmon I, Decaestecker C, et al: Stereotactic comparison among cerebral blood volume, methionine uptake, and histopathology in brain glioma. AJNR Am J Neuroradiol 28:455-61, 2007

Non-Patent Document 5: LeCun Y, Bengio Y, Hinton G: Deep learning. Nature 521:436-44, 2015

Non-Patent Document 6: Hinton G: Deep Learning-A Technology With the Potential to Transform Health Care. JAMA 320:1101-1102, 2018

Non-Patent Document 7: Mirza M, Osindero S: Conditional Generative Adversarial Nets, 2014, pp arXiv:1411.1784

Non-Patent Document 8: Ueda D, Shimazaki A, Miki Y: Technical and clinical overview of deep learning in radiology. Jpn J Radiol 37:15-33, 2019

Non-Patent Document 9: Mirza M, Osindero S: Conditional Generative Adversarial Nets. arXiv e-prints:arXiv:1411.1784, 2014

Non-Patent Document 10: Armanious K, Jiang C, Fischer M, et al: MedGAN: Medical image translation using GANs. Comput Med Imaging Graph 79:101684, 2020

Non-Patent Document 11: Bazangani F, Richard FJP, Ghattas B, et al: FDG-PET to T1 Weighted MRI Translation with 3D Elicit Generative Adversarial Network (E-GAN). Sensors (Basel) 22, 2022

SUMMARY OF THE INVENTION

Problem to be solved by the invention

In light of the potential correlation between the magnetic resonance (MR) image and the positron emission tomography (PET) image, the inventors have thought that the image-to-image translation technique could be used to generate a PET-like image from the MR image of the patient, have reached the present invention. Here, the target disease is not limited to the glioma, but may be other tumors or even other diseases. Further, the patient is not limited to a human being but may be an animal. Furthermore, the image is not limited to the $^{11}$C-Methionine PET image, and may be any other type of PET image as long as being able to be correlated with the MR image.

That is, an object of the present invention is to provide a method, device and program for generating a synthetic image which is similar to a positron emission tomography (PET) image from a magnetic resonance (MR) image of a patient, which includes learning an artificial intelligence model for generating the synthetic image.

MEANS TO SOLVE THE PROBLEMS

In order to solve the above-mentioned problems, a first aspect of the present invention provides a medical image generation method characterized by causing execution of the steps of:

acquiring a magnetic resonance (MR) image of a particular patient suspected of having a certain disease; and generating, from the MR image of the particular patient, a synthetic image similar to a positron emission tomography (PET) image, by using a learned model obtained by learning an artificial intelligence model by using, as learning data, MR images of patients diagnosed to have the disease and the PET images corresponding to the MR images.

In the medical image generation method of the present invention, it is preferable that the artificial intelligence model includes a generator that generates a synthetic image from an input MR image, and a discriminator that accepts a set of the input MR image and the generated synthetic image, or a set of the input MR image and a PET image corresponding to the input MR image, and determines whether the image is true or false.

Further, in the medical image generation method of the present invention, it is preferable that the learning data is composed of a set of a plurality of slices in the MR image and one slice in the PET image corresponding to the MR image.

Further, in the medical image generation method of the present invention, it is preferable that the learning data is composed as a pair of an entire MR image and a cropped image in which an area of lesion is cropped from the entire MR image.

Further, a second aspect of the present invention provides a program for causing execution of the steps of:

acquiring a magnetic resonance (MR) image of a particular patient suspected of having a certain disease; and generating, from the MR image of the particular patient, a synthetic image similar to a positron emission tomography (PET) image, by using a learned model obtained by learning an artificial intelligence model by using, as learning data, MR images of patients diagnosed to have the disease and the PET images corresponding to the MR images.

A third aspect of the present invention provides a medical image generation device comprising:

an acquisition unit that acquires a magnetic resonance (MR) image of a particular patient suspected of having a certain disease;
a storage unit that stores a learned model obtained by learning an artificial intelligence model by using, as learning data, MR images of patients diagnosed to have the disease and the positron emission tomography (PET) images corresponding to the MR images; and
an image generation unit that generates a synthetic image similar to the PET image from the MR image of the particular patient by using the learned model.

A fourth aspect of the present invention provides an artificial intelligence model learning method comprising the steps of:

acquiring magnetic resonance (MR) images of a patient group diagnosed with a specific disease and positron emission tomography (PET) images corresponding to the MR images; and
learning an artificial intelligence model by using the acquired MR images and PET images as learning data to generate a learned model that generates a synthetic image similar to the PET image from the MR images of patients suspected of having the disease.

A fifth aspect of the present invention provides a program for executing the steps of:

acquiring magnetic resonance (MR) images of a patient group diagnosed with a specific disease and positron emission tomography (PET) images corresponding to the MR images; and

learning an artificial intelligence model by using the acquired MR images and PET images as learning data to generate a learned model that generates a synthetic image similar to the PET image from the MR images of patients suspected of having the disease.

A sixth aspect of the present invention provides an artificial intelligence model learning device comprising:

a storage unit that stores magnetic resonance (MR) images of a patient group diagnosed with a specific disease and positron emission tomography (PET) images corresponding to the MR images; and

a learning unit that learns an artificial intelligence model by using the acquired MR images and PET images as learning data to generate a learned model that generates a synthetic image similar to the PET image from the MR images of patients suspected of having the disease.

Here, each of the above methods is a computer-implemented method. Further, each of the above programs may be stored in a non-transitory computer-readable storage medium and executed by a processor.

EFFECT OF THE INVENTION

According to the present invention, a synthetic image similar to a PET image can be generated from an MR image of a patient. This makes it possible to provide a diagnosis equivalent to that of a PET examination to a patient who has only undergone an MR examination. Thus, it is possible to provide a more sufficient diagnostic data to the patient who is under consleaned resources.

BRIEF EXPLANATION OF DRWAINGS

FIG. 1 is a block diagram showing the software configuration of the medical image generation device 10.

FIG. 2 is a flowchart showing the procedures for generating the medical image.

FIG. 3 is a block diagram showing the functional configuration of the artificial intelligence model learning device 20.

FIG. 4 is a flowchart showing the learning procedures of the artificial intelligence model.

FIG. 5 is a block diagram showing the hardware configuration of a computer that constitutes the medical image generation device 10 and the artificial intelligence model learning device 20.

FIG. 6 is a diagram illustrating an outline of the learning model.

FIG. 7 is a diagram showing an example of registration of a Positron Emission Tomography (PET)-Magnetic Resonance (MR) image pair.

FIG. 8 is a diagram showing an example of relative value display for each pixel.

FIG. 9 is a diagram showing an example of a pair of three MR slices and one PET slice.

FIG. 10 is a diagram showing an example of creating a cropped lesion image.

FIG. 11 is a diagram showing an example of a CE-MR image, a synthetic MET-PET image, and an actual MET-PET image.

FIG. 12 is graphs showing the results of the receiver operating characteristic (ROC) analysis and the overall survival (OS) analysis.

FIG. 13 is an explanatory diagram of the U-Net.

MODE FOR CARRYING OUT THE INVENTION

In the following, by referring the drawings, the typical embodiments according to the present invention are explained in detail. However, the present invention is not particularly limited to the drawings. Further, since these drawings are presented to explain the concept of the present invention, there are cases where sizes, ratios and numbers are exaggerated or simplified as necessary for ease of understanding.

1. Medical image generation device 10 and method

The medical image generation device 10 is a computer which generates a synthetic image similar to a positron emission tomography (PET) image (hereinafter sometimes referred to as a synthetic PET image) from a magnetic resonance (MR) image of a patient. The medical image generation device 10 may be a single computer, or may be configured as a system including multiple computers.

In this embodiment, the patient is assumed to be a human, but the patient may also be an animal. Further, the anticipated diseases include tumors, particularly brain tumors, but are not limited to these, and the present invention can also be applied to other diseases such as epilepsy and Alzheimer's.

The MR image is assumed to be a contrast-enhanced T1-weighted (CE-MR) image, but is not limited thereto, and, for example, a simple T1-weighted image, a T2-weighted image, or a diffusion-weighted image can also be used.

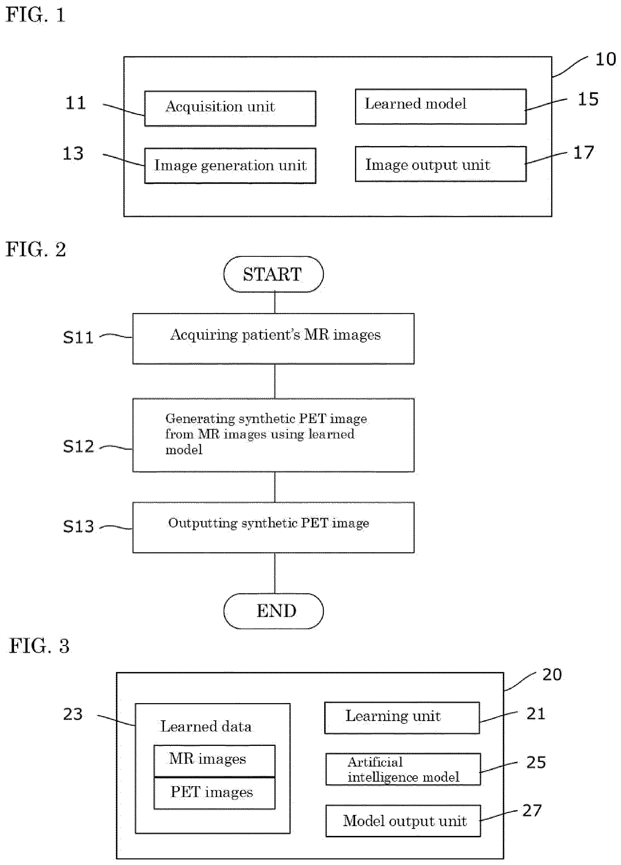

As shown in FIG. 1, the medical image generation device 10 includes an acquisition unit 11, an image generation unit 13, a learned model storage unit 15, and an image output unit 17.

The acquisition unit 11 acquires the MR images of a specific patient. The acquisition unit 11 may be an MR imaging device that generates MR images, or may be a memory that receives and stores the MR images from the MR imaging device or an external device via a communication interface.

The image generation unit 13 generates a synthetic PET image from the MR image of the specific patient by using the learned model stored in the learned model storage unit 15. Here, the learned model is obtained by learning an artificial intelligence model by using the MR image of the patient group and the PET image corresponding to the MR image as learning data. Details of the artificial intelligence model and the learning will be described later.

The image output unit 17 transmits the generated synthetic PET image to an external device, prints, or displays on a display.

With reference to FIG. 2, the general procedures for generating the medical image will be described. Such procedures may be carried out by a computer which functions as the medical image generation device 10.

In step S11, the MR image of the particular patient suspected of having a certain disease is acquired. The step S11 may be executed by the acquisition unit 11 described above.

In step S12, the synthetic PET image is generated from the acquired MR images by using the learned model. The step S12 may be executed by the image generation unit 13 described above.

In step S13, the generated synthetic PET image is output. The step S13 may be executed by the image output unit 17 described above.

The synthetic PET image may be used by a medical doctor to examine and diagnose the patient. The synthetic PET images are especially useful when a PET machine is not available.

2. Artificial intelligence model learning device 20 and method

The artificial intelligence model learning device 20 is a computer that generates the learned model, and may be a single computer or may be configured as a system including multiple computers. The artificial intelligence model learning device 20 may be a single computer integrated with the medical image generation device 10, or may be another computer being different from the medical image generation device 10.

As shown in FIG. 3, the artificial intelligence model learning device 20 includes a learning unit 21, a learning data storage unit 23, an artificial intelligence model storage unit 25, and a model output unit 27.

The learning unit 21 learns the artificial intelligence model by using the learning data and generates a learned model. In this embodiment, among deep learning techniques, the generative adversarial network (GAN), which is a type of generative model, is preferably used, but other generative models such as the variational autoencoder (VAE) or the diffusion model may be used as long as they are capable of generating the PET-like image from the MR image.

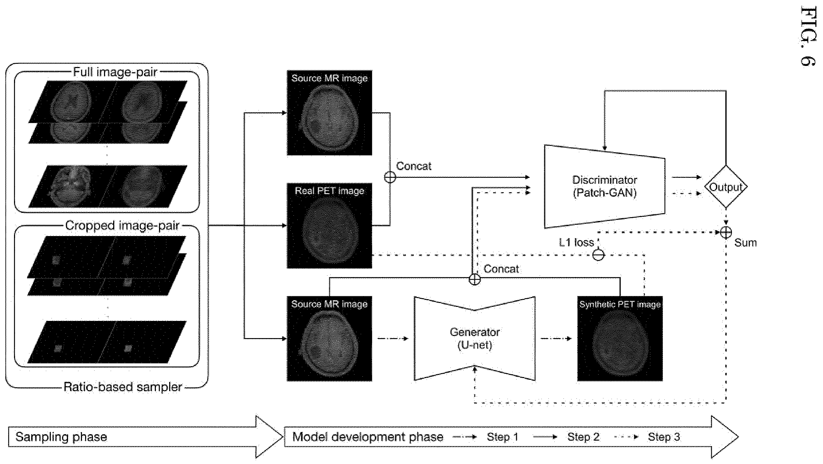

Here, the GAN comprises the generator and the discriminator. The generator generates a synthetic image similar to the PET image from the MR images. The discriminator receives the synthetic image generated by the generator and the real PET image and determines which is real or false (see FIG. 6). By alternately learning these two networks in competition, the generator is able to generate a synthetic image that is closer to the real PET image.

More specifically, the competitive relationship between the two networks is expressed by sharing the loss function L expressed by the following equation. That is, for a pair of images (x, y), the generator G (which generates an image G(x) from an image x) is learned to reduce the value of the loss function L, and the discriminator D is learned to increase the value of the loss function L.

[Equation 1]

$$L(G,D) = \mathbb{E}_{x,y}[\log D(x,y)] + \mathbb{E}_x[\log (1 - D(x, G(x)))]$$

Provided that, $D(x,y) = \begin{cases} 1 & if\ image\ x\ and\ image\ y\ are\ both\ true \\ 0 & otherwise \end{cases}$

In this embodiment, among the GANs, pix2pix which is a model for converting features between images, is preferably used, but the present invention is not limited thereto. When the pix2pix model is used, the learning of the G is conducted by minimizing the following equation, which takes into account the L1 distance in the loss function L(G,D), so that image G(x) is as close as possible to image x.

$$[\text{Equation 2}]$$
$$L(G,D) + \lambda \mathbb{E}_{x,y}[\|y - G(x)\|_1]$$

The learning data storage unit 23 stores the learning data. The learning data is a set of the MR image and the PET image of the patients diagnosed with a certain disease, and is preferably pre-processed. As the main pre-processing, there may be treated a process of matching multiple slices of the MR image with one slice of the PET image, and a process of cutting out or trimming only the lesion area from the full-size MR image and PET image (full image) to generate a cropped image. The MR image corresponding to one slice of the PET image preferably consists of two or more consecutive slices, and more preferably consists of three or more consecutive slices. The details of the pretreatment will be described later. Note that, the "slice" refers to a cross section of an arbitrary region in three-dimensional data obtained by MR scanning or PET scanning.

The artificial intelligence model storage unit 25 stores the artificial intelligence model and the learned model.

The model output unit 27 stores the generated learned model in an internal or external storage medium, or transmits to an external computer such as the medical image generation device 10.

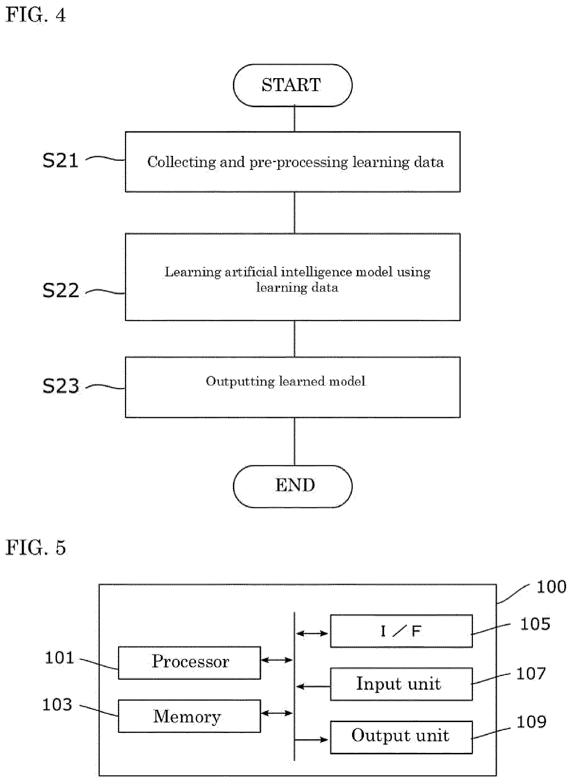

The general procedures for learning the artificial intelligence model will be described with reference to FIG. 4. Such procedures may be carried out by a computer functioning as the artificial intelligence model learning device 20.

In step S21, the MR images and PET images of a patient are collected and registered as the learning data. The step S21 can be realized by the learning data storage unit 23. Any necessary pre-processing may be performed before the images are registered. The main pre-processing is as described above, other pre-processing may be image resizing, reslicing and normalization.

In step S22, the artificial intelligence model is learned by using the registered learning data to obtain the learned model. The step S22 can be realized by the learning unit 21.

In step S23, the obtained learned model may be stored and transmitted to another device, such as the medical image generation device 10. The step S23 can be realized by the artificial intelligence model storage unit 25 and the model output unit 27.

3. Computer constituting the medical image generation device 10 and the artificial intelligence model learning device 20

FIG. 5 shows an example of the hardware configuration of the computer 100 which constitutes the medical image generation device 10 and the artificial intelligence model learning device 20. As shown, the computer 100 includes a processor 101, memory 103, and a communication interface 105, and may also include an input device 107 and an output device 109.

The processor 101 realizes various functions of the medical image generation device 10 and the artificial intelligence model learning device 20 by reading various programs and data into the memory 103 and executing them. The processor 101 may be configured as a semiconductor integrated circuit such as a central processing unit (CPU), a graphics processing unit (GPU), or a microprocessor.

The memory 103 is a random access memory (RAM) and a read only memory (ROM), which stores various data and programs. The memory 103 is a non-transitory computer-readable storage medium, and includes, for example, a hard disk drive, a solid-state drive, or a flash memory.

The communication interface 105 is an interface for connecting to wired and wireless communication networks, and is, for example, an adapter for connecting to Ethernet (registered trademark), a modem for connecting to a public telephone line network, a wireless communication device for wireless communication, a USB (Universal Serial Bus) connector or an RS232C connector for serial communication, or the like.

The input device 107 inputs various types of data, and is, for example, a keyboard, a mouse, a touch panel, a button, a microphone, or the like.

The output device 109 outputs various types of data, and is, for example, a display, a printer, a speaker, or the like. The output device 109 may transmit various data to an external computer or external device via the communication interface 105.

4. Effect of this embodiment

According to this embodiment, the synthetic image similar to the PET image can be generated from the MR image of the patient.

The PET examination is resource intensive in terms of requiring a cyclotron, a PET scanner, medical staff and period of time, which can lead to high medical costs. Furthermore, the PET examination involves exposure of the patient to radiation. In contrast, the synthetic PET image can be obtained from the more popular MR image without the above-mentioned inconveniences and additional examinations. Therefore, when the PET examination is not available, the synthetic PET image is expected to facilitate the differentiation of diseases such as brain tumors and prognosis prediction.

5. Example

An example of this embodiment will be described with reference to FIG. 6 to FIG. 13.

Here, there is provided an illustrative example of a scene in which machine learning is used to generate a PET-like synthetic image from a contrast-enhanced T1-weighted (CE-MR) image obtained by an MR examination performed on a patient suspected of having a glioma. However, this technique is not limited to the diagnosis of glioma, but can also be applied to the diagnosis of tumors other than glioma, and further, other diseases.

Further, as the learning data, a $^{11}$C-Methionine PET (MET-PET) image is used together with the contrast T1-weighted image, but other PET images such as a $^{18}$F-fluorodeoxyglucose (FDG) PET image may also be used.

5-1. Summary of the example

The inventors collected data from the patients who underwent both the MET-PET examination and the CE-MR examination. The MET-PET image and the CE-MR image from the same patient were paired. The group of these image pairs were used to train, validate, and test the image-to-image translation AI model. Then the external validation of the learned model was performed to show the performance in grading and predicting prognosis of the synthetic MET-PET images generated from the CE-MR images of glioma patients.

In the following, the example will be described in detail.

5-2. Deep learning and dataset

The deep learning has three main phases, i.e. training, validation, and testing. It is common to prepare training and validation datasets for training and adjustment the AI model. The dataset for testing is used to test the model. In order to avoid overestimation and overfitting of the model, the same data and images are not used in any two datasets.

The training of the deep learning is usually carried out on randomly sampled batches (i.e., subsets) of the training datasets, and these batches are used to optimize the weight of the model via backpropagation. The training is performed by repeatedly updating the model parameters until the model fits the data optimally.

The validation dataset is used for parameter selection and adjustment, and can also be used to perform the stop condition for training.

In the testing phase, an independent dataset is used to evaluate the discrimination performance of the AI model. Thereby, the generalizability of the model is ensured.

An internal testing dataset is an independent dataset used to evaluate the performance of the model that is not used in the training phase, but is acquired from the same facility as the data used in the training phase. An external dataset for testing is a dataset for performance evaluation that is not used in the training phase and is collected from another facility.

5-3. Preparation of dataset

At first, the AI model was trained to prepare a dataset in the present facility (herein referred to as the internal dataset) for validating and internally testing. Here, the present facility refers to the hospital of the School of Medicine of UNIVERCITY PUBLIC CORPORATION OSAKA.

That is, the patients who underwent both the MET-PET examination and CE-MR examination at the present facility between January 2007 and December 2018 were retrospectively collected. However, patients who met the predefined exclusion criteria were excluded. The pathological diagnosis was determined pathologically if the patient underwent surgery, otherwise the diagnosis it was determined according to the international guidelines for each disease.

The details of the CE-MR examination are as follows.

The patients were administrated intravenously 0.1 ml/kg of gadobutrol (Gadovist; registered trademark) or 0.2 ml/kg of meglumine gadoterate (Magnescope; registered trademark) or gadoteridol (ProHance; registered trademark). The CE-MR images were scanned as routine examinations. The acquired parameters were as follows: echo time 1.7 to 20 msec; repetition time 4.0 to 2020 msec; matrix 256x256 to 640x640; and slice thickness 0.8 to 5 mm.

The details of the MET-PET examination are as follows.

The patients were administered $^{11}$C-Methionine intravenously at 6 MBq/kg over 30 seconds while fasting. After acquiring the transmission scan, a 10-second static scan was initiated 20 minutes after the administration. The acquired parameters were as follows: matrix 128x128 to 336x336; and slice thickness 2.0 to 3.2 mm.

Next, with respect to the leaned model, the data for external validation (herein referred to as an external dataset) was prepared. The external dataset consists of the MR imaging, pathological diagnosis, grading, and prognostic data from the patients diagnosed with primary glioma who underwent the preoperative MR examinations but no MET-PET examinations.

In this example, the following open source datasets were used as the external datasets:

- The Cancer Genome Atlas Low Grade Glioma Collection (http://doi.org/10.7937/K9/TCIA.2016. L4LTD3TK); and
- The Cancer Genome Atlas Glioblastoma Multiforme Collection (https://doi.org/10.7937/K9/TCIA.2016. RNYFUYE9).

These external data sets were collected from multiple facilities in the United States and Italy. However, patients who met the predefined exclusion criteria were excluded.

The details of the MRI examination are as follows.

The type of the contrast agent used was not disclosed and was unknown. The CE-MR images have been scanned with the following acquiing parameters: echo time 1.6 to 20 msec; repetition time 4.7 to 3285 msec; matrix 240x240 to 512x512; and slice thickness 0.9 to 6 mm.

5-4. Distribution of data

All patient data in the internal dataset was randomly assigned into training, validation, and internal testing datasets in a ratio of 8:1:1. Such patient-based distribution ensures that there is no overlap of images or patients between each dataset.

All patients in the external dataset were from different countries than the present facility and were used to demonstrate the generalization of the present AI model.

5-5. Ground truth labeling

In this embodiment, the voxels in the MET-PET images having pixel values 1.3 times or more than the average pixel value of the contralateral cerebral cortex with a normal appearance were defined as lesions.

Two radiologists (blinded to the patient's diagnosis and prognosis) segmented the PET-PET and CE-MR images as follows, and the other radiologist (blinded to the patient's diagnosis and prognosis) turned the images to produce the final segmentation. All regions of interest (ROI) were generated by using the ITK-SNAP.

a) Reference ROI of the cerebral hemisphere for MET-PET image

To normalize the MET-PET image, the signal intensity of the cerebral cortex opposite the lesion with a normal appearance, was taken as the reference. More specifically, five spherical ROIs with a diameter of 1 cm were placed in the contralateral cerebral hemisphere, which appeared normal on the MET-PET image. Alternatively, when the lesion was in both cerebral hemispheres, five spherical ROIs were placed in the normal-appearing cerebral cortex on both sides.

b) Reference ROI of the cerebellar hemisphere for CE-MR image

To normalize the CE-MR images, the signal intensity of the cerebellar white matter with a normal appearance was used as the reference. More specifically, two spherical ROIs with a diameter of 1 cm were placed in the left and right cerebellar white matter on the CE-MR image. Alternatively, when either the left or right cerebellar white matter had the lesion, two ROIs were placed in the cerebellar white matter opposite the lesion.

This process allows MET-PET and CE-MRI to be expressed in relative pixel values, absorbing differences in pixel representation due to differences in image capture equipment and software processing.

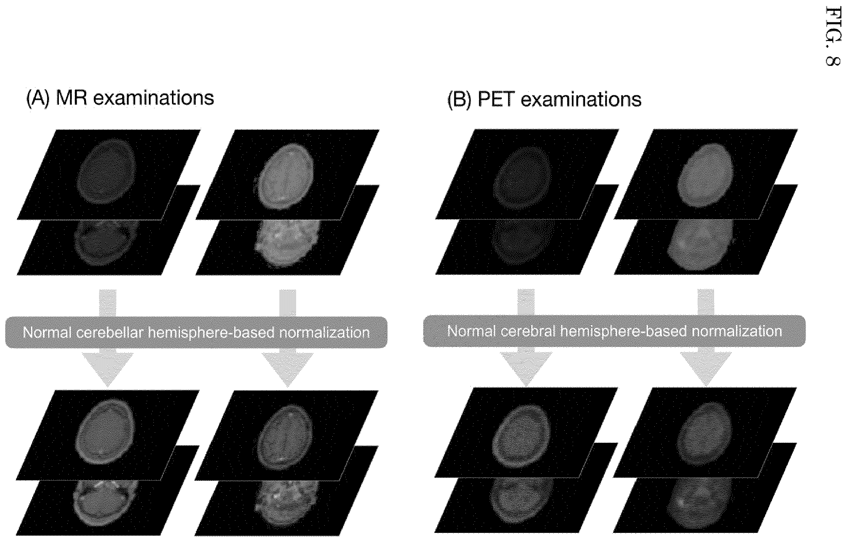

FIG. 8 shows an example of images before and after the normalization. Panels (A) and (B) show MR and PET images, respectively. The upper figure is the original images, and the basis for correcting the between-image differences.

The maximum and mean pixel values of lesions on MET-PET images divided by the mean pixel value of a contralateral cerebral cortex with normal appearance were defined as the standardized uptake value ratio (SUVR) max and SUVR mean, respectively.

5-6. Generating learning model

5-6-1. Preprocessing

In this embodiment, the AI model was developed based on the pix2pix model, that is, a generative adversarial network (GAN) that performs image-to-image translation using extracted features between image-pairs (see FIG. 6). However, this model has two different points from the conventional pix2pix developments: (a) the three-dimensional (3D) data handling method, and (b) the data sampling method. These allow efficient feature learning regarding methionine uptake in lesions.

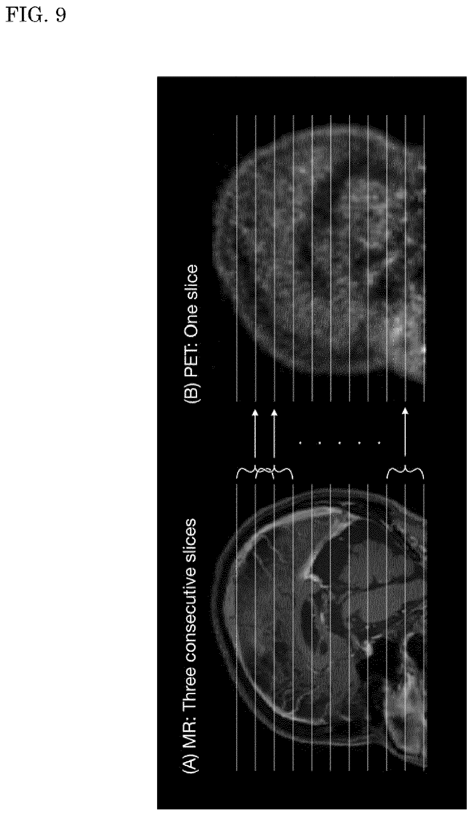

Regarding the above (a), the present model is so designed as to utilize three consecutive slices of the CE-MR images to generate one synthetic MET-PET image. The panels (A) and (B) of FIG. 9 show examples of MR image and PET image, respectively. Three consecutive MR image slices are paired with one PET image slice.

This procedure is a technique to efficiently learn 3D data. That is, the main purpose of typical image-to-image translation models is the conversion task between two-dimensional images, such as generating a map from an aerial photograph, and the typical conversion models are not designed to handle three-dimensional image information. What is handled in this embodiment is the three-dimensional information such as the continuity of a lesion. From this point of view, when creating the paired images, the inventors paired the consecutive slices (n-1, n, n+1) of the CE-MR image with one slice 1(n) of the MET-PET image (where n is a natural number indicating the slice number of the image data).

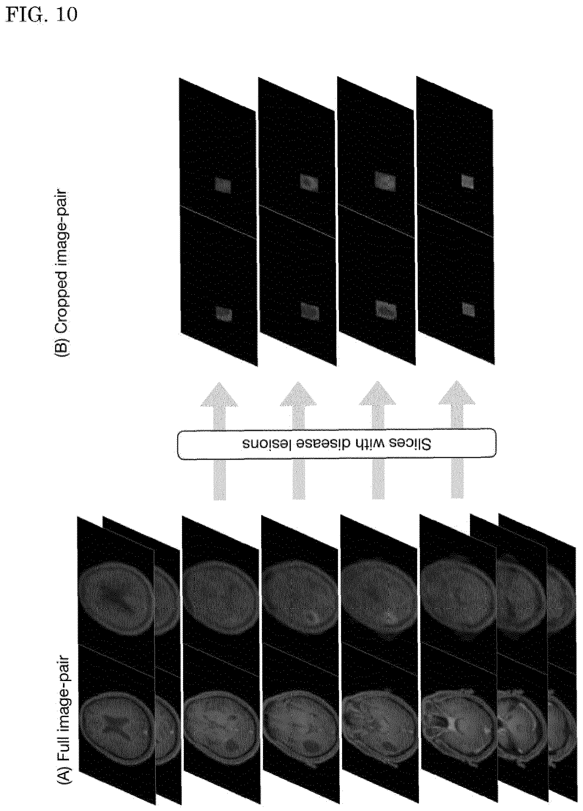

Regarding the above (b), first, two base image pairs, i.e., a cropped image-pair and a full image-pair are prepared. The cropped image-pair comprised three consecutive CE-MR image slices and one MET-PET image slice of only the disease region only (the remaining area outside the cropped area may be padded with black). The full image-pair comprised three consecutive CE-MR image slices and one MET-PET image slice of the full image (i.e., the image that has not been cropped). FIG. 10 shows an example of the cropped image-pair and the full image-pair.

Thus, by using both full and cropped images during the learning process to allow extraction of the features of both normal and abnormal methionine uptake.

The above data sampling techniques (a) and (b) enable the AI model to efficiently learn both local lesion information and information from the entire brain.

Therefore, the registered pairs of CE-MR image and MET-PET image become the base pairs of the learning data. In the registered image pairs, the alignment, slice range, photographing angle, patient orientation, and the like have already been adjusted.

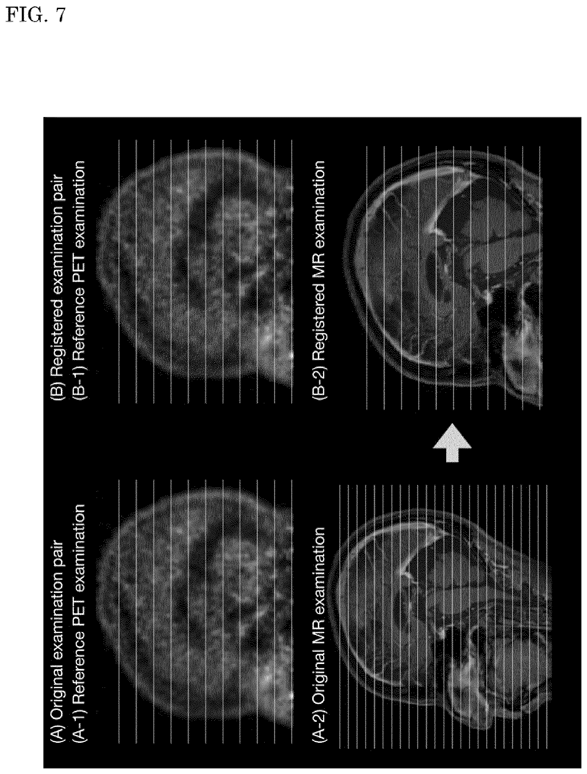

FIG. 7 shows an example of an image before and after registration. Panels (A) show the original pairs before registration. Panel (A-1) shows the reference PET image, while Panel (A-2) shows the original MR image. Panels (B) show converted pairs after registration. Panel (B-1) shows the same image as Panel (A-1), while Panel (B-2) shows the resliced and registered MR image based on the reference PET image (A-1). White lines show the location of axial sliced section.

5-6-2. Learning

After these preprocessing steps, the pix2pix model was trained. The model is trained, tuned, and evaluated on the training, validation, and internal testing datasets, respectively. The entire network is utilized for pixel-wise regression and end-to-end mapping.

FIG. 6 is an explanatory diagram of the learning of deep learning model.

The deep learning model according to this embodiment was developed based on pix2pix, which is a type of generative adversarial network. This is an image-to-image translation model that uses paired images in training and validation datasets. In this deep learning model, a U-Net-based architecture is employed as the generator, and a convolutional PatchGAN discriminator is employed as the discriminator.

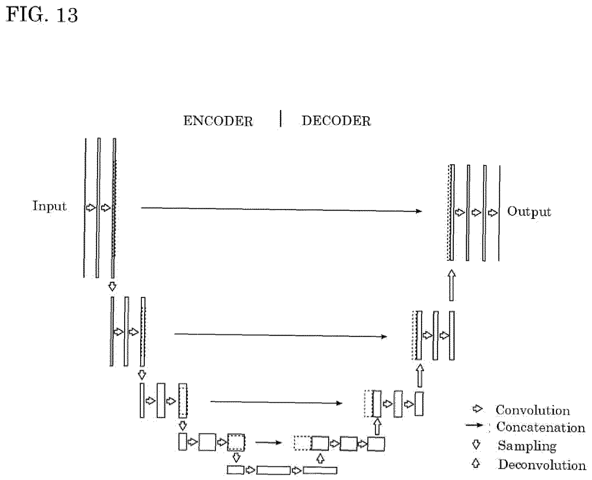

Here, the U-Net is a full-layer convolutional network for image segmentation, and is a model composed of an encoder and a decoder (see FIG. 13). The encoder convolves the input image several times to extract the features from the image. The encoder can utilize the structure of the image classification models such as ResNet. In contrast, the decoder receives the features extracted by the encoder, performs a deconvolution process (upsampling), and outputs a probability map of the same size as the input image. In addition, the U-Net concatenates the feature map of the encoder to the feature map of decoder at each layer. As a result, the information of the large feature map on the encoder side can be transmitted to the decoder side, making it easier to capture the position information during of the object during the upsampling.

The PatchGAN discriminator decomposes the input image into patches of NxN resolution and discriminates each patch as real or fake. Then, the average of the truth values of all the patches is taken as the output of the discriminator.

The model learning involves two phases: the sampling phase and the model development phase.

In the sampling phase, the sampler of the data loader inputs the data from full image-pairs and cropped image-pairs into the model based on the specified ratio. That is, the sampler contributes to efficient learning of the methionine uptake regions by adjusting the ratio of the cropped image-pairs to the full image-pairs.

More specifically, the images used herein are obtained from patients undergoing the MET-PET examination, and most cases have disease lesions in the images. However, the disease lesions are present in a few slices at most, and there are many more slices without disease lesions. Therefore, if the model is trained by using all images, the image-to-image translation model may generate more normalized synthetic MET-PET images, taking into account the normal-enriched class imbalance. Conversely, if the model is trained using only disease images, disease-enriched class imbalance may result in the model generating synthetic MET-PET images without learning normal methionine uptake.

To correct these class imbalances, the inventors devised a sampler to feed images into the image-to-image translation model. This allows for more efficient extraction of features of both diseased lesion and normal tissue. In this embodiment, when feeding data to the image-to-image translation model during training, the ratio of cropped image-pairs to full image-pairs was adjusted from 1:9 to 9:1 (9 ways).

Next, the model development phase is composed of the following three steps:

- Step 1: Image-generation phase;
- Step 2: Discriminator learning phase; and
- Step 3: Generator learning phase.

In step 1, the generator generates one synthetic MET-PET image from three consecutive CE-MR images. Subsequently, the CE-MR images are concatenated with the synthetic MET-PET image (see dashed-dotted line in FIG. 6).

In step 2, the concatenated images of the synthetic MET-PET image and the CE-MR images from step 1 or the concatenated image of the real MET-PET image and the CE-MR images from the training dataset are input to the discriminator (see solid line in FIG. 6). The discriminator is used to correctly classify the synthetic MET-PET image and the real MET-PET image. Therefore, if the discriminator is correct, the loss value is set small, and if the discriminator is wrong, the loss value is set large. The resulting discriminator loss value is back-propagated to the discriminator to update its parameters.

In step 3, the generator is used to generate the synthetic MET-PET image with such a high similarity to the real MET-PET image that is mistakenly recognized by the discriminator (see dashed line in FIG. 6). Therefore, if the discriminator is wrong, the loss value is set small, and if the discriminator is correct, the loss value is set large, respectively. Furthermore, the L1 loss values from the real MET-PET image and the synthetic MET-PET image are obtained. These two losses are combined to generate the loss value of the generator, followed by updating the generator parameters.

By repeating these processes, learning progresses and a learned model is obtained.

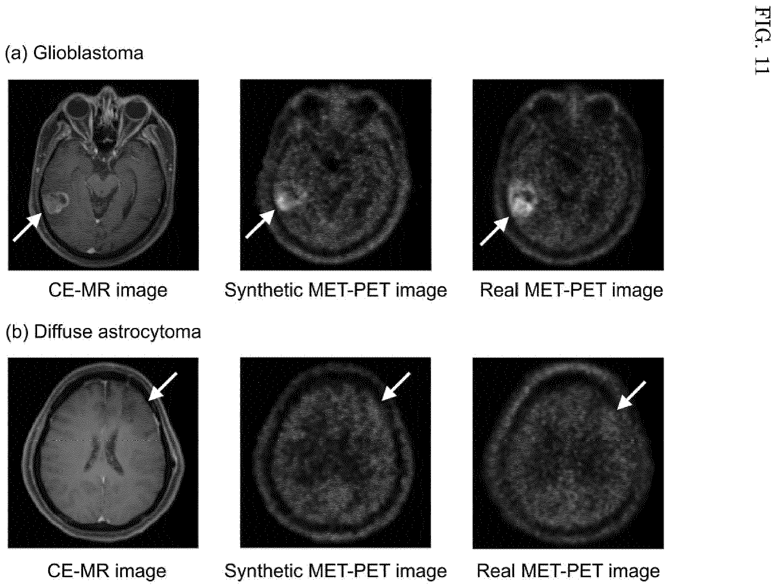

The learned model receives an input of the MR image of a patient and outputs the synthetic image similar to the received MR image. FIG. 11 shows examples of a CE-MR image, a synthetic MET-PET image, and an real MET-PET image of a patient with glioma and diffuse astrocytoma.

5-7. Statistical analysis of generated image

Pearson's correlation coefficients were calculated for the SUVR maximum, SUVR mean, and lesion volume between the synthetic and real MET-PET images.

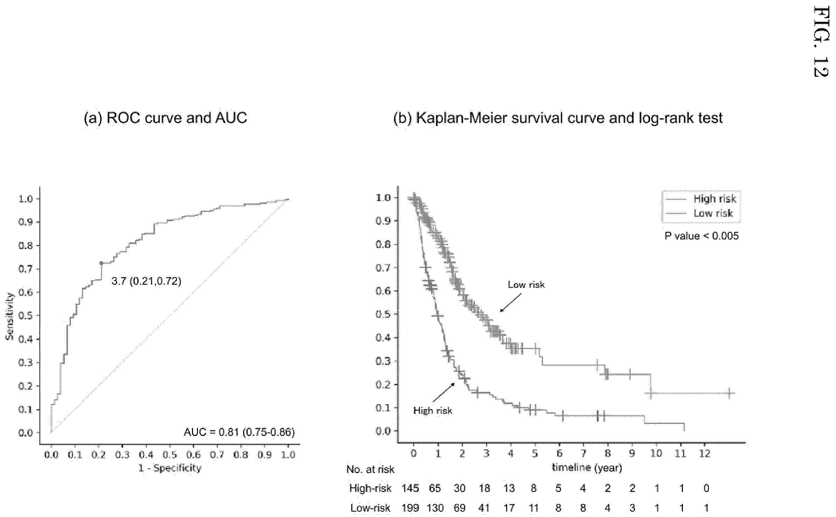

That is, the receiver operating characteristic (ROC) analysis and overall survival (OS) analysis were performed by using the external test dataset. FIG. 12 is a graph showing the results. In the ROC analysis, the area under the curve (AUC) was calculated using the SUVR maximum and the SUVR mean of the synthetic MET-PET images to estimate the accuracy of distinguishing between low-grade and high-grade gliomas. The OS analysis was performed using the Kaplan-Meier method to stratify patients into high-risk and low-risk groups based on the average of the SUVR maximum and the SUVR mean. Stratification performance was assessed using the log-rank test. The OS is defined as the time between the date of glioma diagnosis and the date of death. When the death of a patient is not able to be confirmed, the patient is defined as censored. The follow-up period for censored patients is the time between the date of glioma diagnosis and the date of last contact. Furthermore, the SUVR maximum and SUVR mean in the high-grade and low-grade gliomas for both internal and external testing datasets were analyzed. Statistical significance is set at $P < 0.05$.

5-7-1. Demographic characteristics

As the internal dataset, the 32,075 image-pairs of 475 MET-PET and CE-MR examinations in 362 patients were collected. The training dataset includes 25,848 image-pairs of 384 examinations in 294 patients (mean age $\pm$ standard deviation [SD], 48 years $\pm$ 18; 44% men). The validation dataset includes 3099 image-pairs of 46 examinations in 34 patients (mean age $\pm$ SD, 54 years $\pm$ 17; 52% men). The internal test dataset includes 3128 image-pairs of 45 examinations in 34 patients (mean age $\pm$ standard deviation [SD], 50 years $\pm$ 17; 50% men). The external dataset includes 41,755 images of 344 CE-MR examinations in 344 patients (mean age $\pm$ standard deviation [SD], 53 years $\pm$ 15; 55% men) from two open datasets of The Cancer Imaging Archive.

5-7-2. Model Development Results

The Pearson's correlation coefficients between the synthetic and real MET-PET images were 0.68 (95% confidence interval [CI]: 0.47 to 0.81) for SUVR maximum, 0.76 (95% CI: 0.59 to 0.86) for SUVR mean, and 0.92 (95% CI: 0.85 to 0.95) for lesion volume, with all P values less than 0.001. Generally, the value of the Pearson's correlation coefficient ranges from -1 to 1, with absolute values of 0 to 0.19 indicating very weak correlation, 0.2 to 0.39 indicating weak correlation, 0.40 to 0.59 indicating moderate correlation, 0.6 to 0.79 indicating strong correlation, and 0.8 to 1 indicating very strong correlation, respectively.

5-7-3. Model evaluation in the external cohort results

The ROC for differentiation between low-grade and high-grade gliomas was 0.81 (95% CI: 0.75 to 0.86) for SUVR maximum and 0.78 (95% CI: 0.73 to 0.84) for SUVR mean. Further, the group with SUVR mean values higher than 1.76 had a significantly shorter OS than the group with lower values (P < 0.005).

5-7-4. Evaluation

The inventors have developed the artificial intelligence model to generate the synthetic PET images from the CE-MR images.

In the internal test dataset, Pearson's correlation coefficients revealed strong to very strong correlations between the synthetic and real MET-PET images for SUVR maximum, SUVR mean, and lesion volume. Regarding the differentiation between low-grade and high-grade gliomas, the ROC analysis of SUVR maximum and SUVR mean showed high AUC. The group with SUVR maximum and SUVR mean values higher than the average had significantly shorter OS than the group with values lower than the average. These results show that the synthetic PET images generated from the CE-MR images are clinically useful.

To the inventors' knowledge, this is the first example of generating the synthetic PET images from the MR images. The MRI examination is usually used for static evaluations from the viewpoints of being suitable for evaluating the morphology and features of organs. In contrast, the PET examination can be used for dynamic evaluation of the metabolic state of lesions. In this respect, the MR and PET examinations are complementary.

The inventors have successfully generated the PET images which are the dynamic examination images, from the MR images which are the static examination images. This suggests that the MR images may contain metabolic information of lesions.

The PET examination is resource-intensive in terms of requiring a cyclotron, PET scanner, medical staff and time, which leads to high medical costs. Furthermore, the examination involves exposing the patient to radiation. In contrast, the synthetic PET images can be obtained from the MR images without the above-mentioned inconveniences and additional examinations. This is because the MR images are generally obtained in routine clinical situations for patients with suspected brain tumors. In case that the PET examination is not feasible, this model will facilitate the differentiation of brain tumors and prognosis prediction.

In conclusion, this artificial intelligence model enables the generation of the synthetic PET images from the MR images. The synthetic PET images are strongly correlated with the real PET images and showed significant performance for grading and prognosis of gliomas. The present artificial intelligence model can generate clinically useful images without the radiation exposure from the MR images, which may be useful for patients with gliomas and the like, especially in resource-limited situations.

In the above, although the typical embodiments of the present invention have been described, the present invention is not limited to these, and various design changes are possible, and all of those are included in the present invention.

Explanation of Symbols

10 Medical image generation device
11 Acquisition unit
13 Image generation unit
15 Learned model storage unit
17 Image output unit
20 Artificial intelligence model learning device
21 Learning unit

23 Learned data storage unit
25 Artificial intelligence model storage unit
27 Model output unit

**Claims**

1. A medical image generation method **characterized by** causing execution of the steps of:

   acquiring a magnetic resonance (MR) image of a particular patient suspected of having a certain disease; and generating, from the MR image of the particular patient, a synthetic image similar to a positron emission tomography (PET) image, by using a learned model obtained by learning an artificial intelligence model by using, as learning data, MR images of patients diagnosed to have the disease and the PET images corresponding to the MR images.

2. The medical image generation method according to claim 1, wherein

   the artificial intelligence model includes a generator that generates a synthetic image from an input MR image, and
   a discriminator that accepts a set of the input MR image and the generated synthetic image, or a set of the input MR image and a PET image corresponding to the input MR image, and determines whether the image is true or false.

3. The medical image generation method according to claim 2, wherein
   the learning data is composed of a set of a plurality of slices in the MR image and one slice in the PET image corresponding to the MR image.

4. The medical image generation method according to claim 2, wherein
   the learning data is composed as a pair of an entire MR image and a cropped image in which an area of lesion is cropped from the entire MR image.

5. A program for causing execution of the steps of:

   acquiring a magnetic resonance (MR) image of a particular patient suspected of having a certain disease; and generating, from the MR image of the particular patient, a synthetic image similar to a positron emission tomography (PET) image, by using a learned model obtained by learning an artificial intelligence model by using, as learning data, MR images of patients diagnosed to have the disease and the PET images corresponding to the MR images.

6. A medical image generation device comprising:

   an acquisition unit that acquires a magnetic resonance (MR) image of a particular patient suspected of having a certain disease;
   a storage unit that stores a learned model obtained by learning an artificial intelligence model by using, as learning data, MR images of patients diagnosed to have the disease and the positron emission tomography (PET) images corresponding to the MR images; and
   an image generation unit that generates a synthetic image similar to the PET image from the MR image of the particular patient by using the learned model.

7. An artificial intelligence model learning method comprising the steps of:

   acquiring magnetic resonance (MR) images of a patient group diagnosed with a specific disease and positron emission tomography (PET) images corresponding to the MR images; and
   learning an artificial intelligence model by using the acquired MR images and PET images as learning data to generate a learned model that generates a synthetic image similar to the PET image from the MR images of patients suspected of having the disease.

8. A program for executing the steps of:

acquiring magnetic resonance (MR) images of a patient group diagnosed with a specific disease and positron emission tomography (PET) images corresponding to the MR images; and

learning an artificial intelligence model by using the acquired MR images and PET images as learning data to generate a learned model that generates a synthetic image similar to the PET image from the MR images of patients suspected of having the disease.

9.   An artificial intelligence model learning device comprising:

a storage unit that stores magnetic resonance (MR) images of a patient group diagnosed with a specific disease and positron emission tomography (PET) images corresponding to the MR images; and

a learning unit that learns an artificial intelligence model by using the acquired MR images and PET images as learning data to generate a learned model that generates a synthetic image similar to the PET image from the MR images of patients suspected of having the disease.

FIG. 1

```
                                                              10
  11      Acquisition unit           Learned model         15

  13      Image generation unit      Image output unit      17
```

FIG. 2

START

S11 — Acquiring patient's MR images

S12 — Generating synthetic PET image from MR images using learned model

S13 — Outputting synthetic PET image

END

FIG. 3

```
                                                              20
                                      Learning unit          21
  23      Learned data
                                      Artificial
            MR images                 intelligence model     25

            PET images                Model output unit      27
```

FIG. 4

```
                    ┌─────────────┐
                    │    START    │
                    └──────┬──────┘
                           │
       ┌─────────────────────────────────────────────┐
S21 ───┤   Collecting and pre-processing learning data │
       └─────────────────────────────────────────────┘
                           │
       ┌─────────────────────────────────────────────┐
       │   Learning artificial intelligence model using│
S22 ───┤              learning data                    │
       └─────────────────────────────────────────────┘
                           │
       ┌─────────────────────────────────────────────┐
S23 ───┤           Outputting learned model            │
       └─────────────────────────────────────────────┘
                           │
                    ┌─────────────┐
                    │     END     │
                    └─────────────┘
```

FIG. 5

```
                                              ┌──────── 100
   ┌──────────────────────────────────────────────────┐
   │                              ┌──────────────┐     │
   │                         ┌───▶│     I / F     │────── 105
   │   ┌──────────────┐      │    └──────────────┘     │
101─┤──│  Processor    │◀────┤                          │
   │   └──────────────┘      │    ┌──────────────┐     │
   │                         ◀────│  Input unit   │────── 107
   │   ┌──────────────┐      │    └──────────────┘     │
103─┤──│   Memory      │◀────┤                          │
   │   └──────────────┘      │    ┌──────────────┐     │
   │                         └───▶│  Output unit  │────── 109
   │                              └──────────────┘     │
   └──────────────────────────────────────────────────┘
```

FIG. 6

FIG. 7

(A) Original examination pair
(A-1) Reference PET examination
(A-2) Original MR examination

(B) Registered examination pair
(B-1) Reference PET examination
(B-2) Registered MR examination

FIG. 8

EP 4 663 115 A1

(A) MR examinations

(B) PET examinations

Normal cerebellar hemisphere-based normalization

Normal cerebral hemisphere-based normalization

FIG. 9

(A) MR: Three consecutive slices

(B) PET: One slice

FIG. 10

(A) Full image-pair

(B) Cropped image-pair

Slices with disease lesions

FIG. 11

(a) Glioblastoma

CE-MR image — Synthetic MET-PET image — Real MET-PET image

(b) Diffuse astrocytoma

CE-MR image — Synthetic MET-PET image — Real MET-PET image

FIG. 12

(a) ROC curve and AUC

(b) Kaplan-Meier survival curve and log-rank test

FIG. 13

## INTERNATIONAL SEARCH REPORT

| International application No. |
| --- |
| **PCT/JP2024/004071** |

### A. CLASSIFICATION OF SUBJECT MATTER

*A61B 5/055*(2006.01)i; *G06T 1/00*(2006.01)i; *G06T 1/40*(2006.01)i
FI: A61B5/055 380; A61B5/055 383; G06T1/40; G06T1/00 290C

According to International Patent Classification (IPC) or to both national classification and IPC

### B. FIELDS SEARCHED

Minimum documentation searched (classification system followed by classification symbols)

A61B5/055; A61B6/03; G06T1/00-19/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JUNG, Merel M. et al. Inferring PET from MRI with pix2pix, ResearchGate, [online], November 2018, [retrieved on 02 April 2024], pp. 1-9, Internet: <URL: https://www.researchgate.net/profile/Merel-Jung/publication/328788803_Inferring_PET_from_MRI_with_pix2pix/links/5be2f4974585150b2ba580c4/Inferring-PET-from-MRI-with-pix2pix.pdf> p. 2, lines 21-32, p. 3, line 1 to p. 4, line 29 | 1-2, 5-9 |
| A | entire text, all drawings | 3-4 |
| A | SIKKA, Apoorva et al. MRI to PET Cross-Modality Translation using Globally and Locally Aware GAN (GLA-GAN) for Multi-Modal Diagnosis of Alzheimer's Disease, arXive, [online], 01 April 2021, [retrieved on 02 April 2024], 2108.02160v1, pp. 1-12, Internet: <URL: https://doi.org/10.48550/arXiv.2108.02160>, entire text, all drawings | 1-9 |
| A | JP 2020-171841 A (SPLINK INC.) 22 October 2020 (2020-10-22) entire text, all drawings | 1-9 |

☑ Further documents are listed in the continuation of Box C.  ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **03 April 2024** | **16 April 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**

| International application No. |
| --- |
| **PCT/JP2024/004071** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2020-503075 A (AHN, Young Saem) 30 January 2020 (2020-01-30)<br>entire text, all drawings | 1-9 |

Form PCT/ISA/210 (second sheet) (July 2022)

INTERNATIONAL SEARCH REPORT
Information on patent family members

International application No.

**PCT/JP2024/004071**

| Patent document cited in search report | | | Publication date (day/month/year) | Patent family member(s) | | | Publication date (day/month/year) |
|---|---|---|---|---|---|---|---|
| JP | 2020-171841 | A | 22 October 2020 | US | 2021/0257094 | A1 | |
| | | | | entire text, all drawings | | | |
| | | | | WO | 2020/054803 | A1 | |
| | | | | EP | 3851036 | A4 | |
| | | | | CN | 112702947 | A | |
| JP | 2020-503075 | A | 30 January 2020 | US | 2021/0225491 | A1 | |
| | | | | entire text, all drawings | | | |
| | | | | WO | 2019/098780 | A1 | |
| | | | | KR | 10-2019-0056880 | A | |
| | | | | KR | 10-2020-0057463 | A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LANGEN KJ** ; **MUHLENSIEPEN H** ; **HOLSCHBACH M et al.** Transport mechanisms of 3-[123I]iodo-alpha-methyl-L-tyrosine in a human glioma cell line: comparison with [3H]methyl]-L-methionine. *J Nucl Med*, 2000, vol. 41, 1250-5
- **KRACHT LW** ; **FRIESE M** ; **HERHOLZ K et al.** Methyl-[11C]-l-methionine uptake as measured by positron emission tomography correlates to microvessel density in patients with glioma.. *Eur J Nucl Med Mol Imaging*, 2003, vol. 30, 868-73
- **TYNNINEN O** ; **ARONEN HJ** ; **RUHALA M et al.** MRI enhancement and microvascular density in gliomas.. *Correlation with tumor cell proliferation. Invest Radiol*, 1999, vol. 34, 427-34
- **SADEGHI N** ; **SALMON I** ; **DECAESTECKER C et al.** Stereotactic comparison among cerebral blood volume, methionine uptake, and histopathology in brain glioma.. *AJNR Am J Neuroradiol*, 2007, vol. 28, 455-61
- **LECUN Y** ; **BENGIO Y** ; **HINTON G**. *Deep learning. Nature*, 2015, vol. 521, 436-44
- **HINTON G**. Deep Learning-A Technology With the Potential to Transform Health Care.. *JAMA*, 2018, vol. 320, 1101-1102
- **MIRZA M** ; **OSINDERO S**. Conditional Generative Adversarial Nets. *arXiv:1411.1784*, 2014
- **UEDA D** ; **SHIMAZAKI A** ; **MIKI Y**. Technical and clinical overview of deep learning in radiology.. *Jpn J Radiol*, 2019, vol. 37, 15-33
- **MIRZA M** ; **OSINDERO S**. Conditional Generative Adversarial Nets.. *arXiv e-prints:arXiv:1411.1784*, 2014
- **ARMANIOUS K** ; **JIANG C** ; **FISCHER M et al.** MedGAN: Medical image translation using GANs.. *Comput Med Imaging Graph*, 2020, vol. 79, 101684
- **BAZANGANI F** ; **RICHARD FJP** ; **GHATTAS B et al.** FDG-PET to T1 Weighted MRI Translation with 3D Elicit Generative Adversarial Network (E-GAN).. *Sensors (Basel)*, 2022, vol. 22